Europäisches Patentamt

European Patent Office

Office européen des brevets

(19)

(11) Numéro de publication : **0 413 694 B1**

## (12) FASCICULE DE BREVET EUROPEEN

(45) Date de publication du fascicule du brevet :
**12.05.93 Bulletin 93/19**

(51) Int. Cl.$^5$ : **A61K 31/34**, // (A61K31/34, 31:165)

(21) Numéro de dépôt : **89902794.0**

(22) Date de dépôt : **20.02.89**

(86) Numéro de dépôt international :
**PCT/FR89/00065**

(87) Numéro de publication internationale :
**WO 90/09171 23.08.90 Gazette 90/20**

(54) **PREPARATIONS MEDICAMENTEUSES CARDIO-PROTECTRICES COMPRENANT L'AMIODARONE, UN DERIVE NITRE, NOTAMMENT LE DINITRATE D'ISOSORBIDE ET FACULTATIVEMENT UN BETA-BLOQUEUR.**

Le dossier contient des informations techniques présentées postérieurement au dépôt de la demande et ne figurant pas dans le présent fascicule.

(43) Date de publication de la demande :
**27.02.91 Bulletin 91/09**

(45) Mention de la délivrance du brevet :
**12.05.93 Bulletin 93/19**

(84) Etats contractants désignés :
**AT BE CH DE FR GB IT LI LU NL SE**

(56) Documents cités :
**Dialog, No. 5859645/85105155, M. Pfisterer et al.: "Effect of short and long term administration of amiodarone ischaemia-induced left ventricular dysfunction. Implications for combined antianginal drug therapy"
Dialog, No. 5644342/84140008, F. Follath: "Medikamentenwahl und -Dosierung bei Angina Pectroris"**

(56) Documents cités :
**Chemical Abstracts, vol. 101, 1984,(Columbus, Ohio, US), G.R.Hasegawa et al.: "Visual compatibility of amiodarone hydrochloride injection with other injectable drugs", voir page 299, abstract 235444r.
American Journal of Hospital Pharmacy, vol. 41, Jul.84, pp. 1379-1380, G.R. Hasegawa et al.
Schweiz med. Wschr. 114, suppl. 16, 65-69 (1984), F. Follath et al.
Merckx Index, 11ème.ed., no. 5114, pp. 497 et 6528**

(73) Titulaire : **BALIGADOO, Soorianarain
Centre de Recherches Médicales SSR
Université de Maurice
Moka (MU)**

(72) Inventeur : **BALIGADOO, Soorianarain
Centre de Recherches Médicales SSR
Université de Maurice
Moka (MU)**

(74) Mandataire : **Burtin, Jean-François
Cabinet GEFIB, 59, rue Edouard-Vaillant
F-92300 Levallois-Perret (FR)**

EP 0 413 694 B1

## Description

On décrit ici des préparations pharmaceutiques et un mode d'utilisation de celles-ci qui présentent une action cardio-protectrice utile dans le traitement de l'insuffisance coronaire, dans la prévention de la survenue des infarctus ou de la mort subite.

L'invention est basée sur l'usage conjoint de l'Amiodarone, d'un dérivé nitré et facultativement d'un β-bloqueur.

Dans l'insuffisance coronaire, on observe une fréquence importante de morts subites, particulièrement dans l'insuffisance coronaire aiguë dont le point culminant peut être la survenue de l'infarctus du myocarde.

Certains cas de morts subites sont corrélés à des arythmies consécutives à l'insuffisance coronaire elle-même. Il était donc désirable, en même temps, de diminuer la consommation d'oxygène, d'augmenter l'irrigation du myocarde et, également, d'exercer un effet préventif contre les arythmies.

On a constaté que la thrombose se coronaire induit un phénomène appelé "syndrôme myocardial sourd" consécutif à l'ischémie prolongée du coeur. Ce phénomène empêche l'obtention d'un effet bénéfique optimal consécutif à une reperfusion.

L'utilisation d'un dérivé nitré apparait nécessaire dans les premières heures d'un infarctus. Le demandeur a démontré ici l'utilité de l'emploi d'un dérivé nitré dans le traitement de l'ischémie coronaire aiguë de l'infarctus et dans le traitement de l'ischémie présente dans la bordure d'un infarctus du myocarde et dans la zone menacée par l'infarctus.

Ce travail a démontré l'existence de sous-groupes de sujets pour lesquels, les effets indésirables liés à l'administration d'un dérivé nitré étaient relativement importants. Par conséquent, il serait désirable d'administrer une composition qui supprime ces effets secondaires, qui amplifie l'effet anti-ischémique et qui permet un effet anti-ischémique plus prolongé, tout en réalisant une synergie détectable sur le plan clinique.

La présente invention concerne un procédé pour obtenir une action cardio-protectrice chez des sujets souffrant d'ischémie coronaire et par là même, de réduire la probabilité de survenue d'un infarctus du myocarde chez un tel sujet, qui consiste à administrer à un tel sujet, une quantité suffisante cardio-protectrice desdits agents protecteurs contenant de l'amiodarone en association avec, au moins, un membre du groupe consistant en un vaso-dilatateur coronaire ayant au moins un groupement nitré et, de préférence, un β-bloqueur.

D'une manière appropriée le vaso-dilatateur contenant un groupement nitro est choisi dans le groupe consistant en la trinitrine et le dinitrate d'isosorbide et le β-bloqueur est choisi dans le groupe consistant en l'acébutolol, le metoprolol et l'aténolol.

L'énumération de ces vaso-dilatateurs ou de ces β-bloqueurs n'est pas destinée à être limitative ou déterminante.

Ces agents protecteurs sont destinés à être administrés séparement ou sous une forme associée par au moins une des voies d'administration choisie dans le groupe des procédés consistant essentiellement en la voie orale, sub-linguale, intra-veineuse, sous-cutanée et respiratoire.

D'une manière convenable, le rapport pondérale entre l'amiodarone et le vaso-dilatateur coronaire contenant un groupement nitré est de 100 à 1 à 10 et entre 8 et 25, le dosage administré étant compris entre environ 2 et environ 30 mg/kg de poids corporel/jour.

Dans un mode de réalisation, les agents protecteurs sont administrés pendant les premières 30 min après l'apparition d'une insuffisance cardiaque aiguë et convenablement l'administration est répétée au moins une fois, à des intervalles compris entre 30 et 60 min après la première administration.

La présente invention concerne également des agents cardio-protecteurs destinés à être administrés à un malade souffrant d'ischémie coronaire de façon à réduire la probabilité de la survenue d'un infarctus du myocarde chez un tel malade, qui consiste en une combinaison d'une quantité suffisante cardio-protectrice amiodarone et d'un vaso-dilatateur coronaire renfermant au moins un groupement nitré et un support pharmacologique acceptable.

Dans de tels agents, le rapport pondéral de l'amiodarone par rapport au vaso-dilatateur coronaire contenant un groupement nitré se situe entre 10 et 40 pour 1, d'une manière appropriée, le vaso-dilatateur contenant un groupement nitré est choisi dans le groupe consistant en la trinitrine et le dinitrate d'isosorbide. Ces composés sont mélangés sous une forme appropriée pour l'administration par les voies orale, sub-linguale, intra-veineuse, sous-cutanée ou respiratoire.

Dans un autre mode de réalisation, les agents mentionnés ci-dessus peuvent comprendre de l'amiodarone et un β-bloqueur. D'une manière préférée, le rapport pondéral entre l'amiodarone et le β-bloqueur est de 100 à 8 à 25 et la dose totale administrée est comprise entre environ 2 et environ 30 mg/kg de poids corporel/jour.

La présente invention inclut également le procédé selon lequel les compositions pharmaceutiques comme mentionnées ci-dessus, sont utilisées avec des agents fibrinolytiques tels que le Streptokinase, l'uroquinase, l'éminase, RTPA et les produits semblables.

## ETUDES CLINIQUES

Des essais cliniques ont été effectués pour déterminer l'efficacité de certains agents cardio-protecteurs.

Dans une première série d'essais, les études ont été effectuées sur 50 malades consécutifs atteints d'infarctus du myocarde aigü, de manière à observer les effets de l'administration de dinitrate d'isosorbide par voie intra-veineuse et à des doses variant de 2,5 à 7,5 mg/h .

L'effet de l'amiodarone en intra-veineuse, administré seulement pendant 24 heures a également été étudié sur 50 malades successifs atteints d'infarctus du myocarde à la dose de 10 mg/kg/24 heures.

Dans une seconde série d'essais, les malades ont été traités par l'association simultanée d'amiodarone et de dinitrate d'isosorbide. Les deux médicaments ont été administrés en combinaison durant les premières heures de l'infarctus chez 30 sujets.

Le dinitrate d'isosorbide a été administré en premier à la posologie pré-déterminée de 2 à 10 mg/h de façon à apporter sensiblement une efficacité optimale au dinitrate d'isosorbide préalablement à l'administration intra-veineuse d'amiodarone sans que cela provoque une chute, non désirée, de la pression artérielle.

Au cours de l'analyse, cas par cas, l'existence de sous-groupes de malades ont été notés dans lesquels cette association est particulièrment bénéfique comme le montre l'exemple suivant :
- dans un cas d'infarctus avec une ischémie circonférentielle où des fibrillations ventriculaires survenaient, précédées d'épisodes d'élévation très fréquents et brutaux de la pression artérielle, l'administration de dinitrate d'isosorbide par voie intra-veineuse a entraîné la réduction de l'ischémie constatée à l'E.C.G et une chute dans la fréquence des fibrillations ventriculaires. L'adjonction d'amiodarone a entraîné à peu près la cessation des épisodes de fibrillations ventriculaires et une diminution considérable de l'ischémie observée par l'électro-cardiographie.

Les épisodes d'élévation de la pression artérielle ont cessé et le malade a survécu.

Dans ces conditions, les épisodes de mort subite ont cessé.

La preuve de l'efficacité a été apportée d'une manière définitive dans 4 cas où il ne s'est pas produit d'arythmie concomittante, ce qui est une constatation tout à fait surprenante. On a également observé que l'administration en intra-veineuse d'amiodarone après l'administration préalable de dinitrate d'isosorbide a été associée avec une diminution supplémentaire et importante de l'ischémie comme il a été montré par cartographie précordiale ainsi que par les symptômes cliniques et les autres signes cliniques chez 21 des 30 malades.

Chez 4 malades, on a constaté une disparition complète et surprenante des signes électriques de l'ischémie aiguë du myocarde qui suggère une disparition du processus conduisant à l'infarction. On a observé que du dinitrate d'isosorbide entrainait une survenue rapide des effets alors que l'efficacité de l'amiodarone sur l'ischémie survenait d'une manière nette mais seulement après quelques heures et augmentait avec le temps.

Ces essais ont été renouvelés en sens inverse. L'administration du dinitrate d'isosorbide après administration intra-veineuse d'amiodarone a apporté une diminution tout à fait importante des signes électriques au cours de l'ischémie du myocarde chez 6 des 10 cas cliniques étudiés.

Les signes électriques de l'ischémie cardiaque ont disparu complètement au bout de 15 à 20 min respectivement dans 2 cas après le début de l'administration du dinitrate d'isosorbide aux doses utilisées par le demandeur.

Dans deux observations contrôlées, de l'angor de repos, est apparu, d'une manière très fréquente dans une journée et pouvait être considéré comme de l'angor de Prinzmetal de grande sévérité. Dans ce cas, une perfusion du dinitrate d'isosorbide à la dose moyenne de 6 mg/h a empêché la survenue des crises mais avec renouvellement des douleurs, lorsqu'on diminuait la dose de dinitrate d'isosorbide.

Un essai a par conséquent été entrepris pour administrer de l'amiodarone. On a observé qu'après injection directe de 100 mg d'amiodarone suivi par une perfusion, on obtenait l'arrêt des crises angineuses à la dose moyenne de 2 mg/h de dinitrate d'isosorbide. La douleur réapparaissait associée aux signes électriques d'ischémie à la cessation de la perfusion de dinitrate d'isosorbide.

Cette expérience démontre clairement la synergie chez 2 patients pour lesquels, une réaction de la dose nécessaire de dérivé nitré, a pu être obtenue grâce à l'administration conjointe avec l'amiodarone.

Une étude clinique a été entreprise entre 3 groupes contrôlés de 48 malades :
- un groupe recevant seulement le dinitrate d'isosorbide en premier lieu par voie intra-veineuse puis sous forme de comprimés
- un groupe recevant de l'amiodarone
- un groupe recevant un mélange des deux produits.

On a observé des effets bénéfiques dus à l'association des deux produits, plus importants au moyen de l'analyse de plusieurs paramètres comprenant la mortalité, l'intensité de la douleur à l'infarctus et l'augmentation du segment ST à l'ECG, le nombre de territoires ayant un segment ST élevé sur la cartographie précordiale et la survenue d'une différence ventriculaire gauche. Ces résultats ont confiés l'importance de ces deux médicaments en mélange.

Les expérimentations décrites ci-dessus ont été répétées en utilisant l'amiodarone par voie intra-veineuse et la nitro-glycérine par voie intra-veineuse, administrées dans deux ordres chez deux groupes de 6 malades. Une synergie de cette nouvelle association dans la réduction des signes électriques de l'ischémie a été notée.

La synergie observée avec l'amiodarone et le dinitrate d'isosorbide ainsi que celle de l'amiodarone ou la nitro-glycérine par voie intra-veineuse permet de conclure que ce que d'action est commun à tous les dérivés nitrés en général.

L'intensité de la synergie observée à ces doses est surprenante et peut être due à des mécanismes jusqu'ici inconnus au niveau cellulaire. L'observation -faite par le demandeur- de cette synergie qui n'intervient nettement qu'avec des doses d'amiodarone, peut être considérée comme requiérant, d'une manière importante, de nouvelles études sur la synergie entre l'amiodarone et d'autres médicaments, en vue de réduire l'importance de la dose initiale.

Le demandeur a effectué des études avec un certain nombre de médicaments comprenant le dipyridamole, les agents bloqueurs du calcium et les β-bloqueurs et a observé une nette synergie dans les doses administrées, seulement avec l'amiodarone et un β-bloqueur. Il a nettement été observé dans 2 cas d'angor sévère avec des épisodes fréquents répétés d'angor sévère bien définis cliniquement et éléctriquement, que les épisodes pouvaient être empêchés par administration de doses, soit de dinitrate d'isosorbide 24 mg, soit d'amiodarone 25 mg et soit de propanolol 1 mg. Toutefois l'association d'amiodarone et de β-bloqueur est contre-indiquée ainsi qu'il est mentionné dans les dictionnaires français et les précautions d'emploi en FRANCE pour les docteurs. Cette association n'était donc pas suggérée par l'état de la technique antérieure au présent travail.

Dans une troisième série d'expérimentation, on a recherché les effets de l'association amiodarone/β-bloqueur pour comprendre si l'effet de l'amiodarone est dû uniquement à son action sur les récepteurs β-adrénergiques et pour étudier la gamme de posologie auxquelles la synergie apparait ainsi que les limites des contre-indications. Des doses progressivement croissantes d'amiodarone et d'un β-bloqueur, ont été administrées à des malades ayant un angor chronique stable dans les deux ordres différents.

Les observations suivantes inattendues ont été effectuées :

a) lorsque le β-bloqueur est administré en premier, suivi par l'amiodarone, on constate une efficacité statistiquement supérieure avec le mélange de médicaments par rapport au β-bloqueur uniquement sans effets indésirables significatifs majeurs.

Dans un groupe de 80 malades l'essai a été effectué de la manière suivante :
- on a administré un β-bloqueur pendant une période de 3 semaines suivi de l'administration du β-bloqueur à la moité des sujets et l'administration d'une dose réduite du β-bloqueur en mélange avec l'amiodarone à l'autre moitié, pendant une deuxième période de 6 semaines, en mesurant les paramètres significatifs à 3 semaines d'intervalle. La supériorité de l'association de médicaments a été observée par analyse statistique des symptômes et des paramètres électriques sur l'electro cardiographe ergométrique dans l'ensemble ainsi que sur le (monitoring) à l'ECG dynamique sur 24 heures chez 12 malades. Cela suggère l'existence d'une action pharmacologique ainsi est importante d'un point de vue clinique et statistique et qui est différente d'une action anti-adrénergique. Le β-bloqueur employé a été l'aténolol dans 35 cas, l'acébutolol dans 10 cas, le métoprolol dans 10 cas, le sotalol dans 10 cas, le tertatolol dans 6 cas et le propanolol dans 10 cas.

b) Lorsque l'amiodarone a été administrée en premier lieu, suivi par un β-bloqueur dans 30 cas, des effets supplémentaires importants sur le rythme cardiaque, la pression artérielle et des augmentations parfois modérées dans la condition auriculo-ventriculaire, ont été observés après administration de doses moindres que les doses usuelles de β-bloqueurs. Ces augmentations des effets observés sont survenues rapidement , dès les premières doses de β-bloqueur. La durée des exercices sur les tests de cyclisme a augmenté nettement, d'une manière importante, lorsque l'exercice a été préalablement limité par l'angor et les modifications du segment ST. L'augmentation dans la capacité d'exercice était associée à une intensité plus grande de l'effet anti-ischémique détectable, de la combinaison. Ces effets ont été observés avec l'aténolol, le propanolol et l'acébutolol.

Ainsi, l'association d'amiodarone avec un β-bloqueur, à condition que le sujet reçoive d'abord le β-bloquant et que l'on vérifie que le sujet peut supporter le β-bloquant au niveau cardiaque et que la conduction auriculo-ventriculaire n'a pas augmenté d'une manière anormale, est une observation clinique importante. Il parait ainsi que certains effets indésirables de l'association de l'amiodarone avec un β-bloqueur sont dûs à une augmentation des effets β-bloquants chez certains sujets qui ne peuvent pas supporter des effets β-bloquants importants.

L'expérimentation d'un β-bloqueur chez un sujet qui ne présente pas une sensibilité anormale aux β-bloqueurs permet l'utilisation d'une telle association d'un β-bloqueur et d'amiodarone aux doses définies par le Demandeur.

L'augmentation de la durée de conduction qui est observée avec les premières doses de β-bloqueur n'augmente pas davantage avec le temps en maintenant des doses de chacun des médicaments. On peut en tirer la conclusion que les effets hautement bénéfiques de la dite association sont en général correlés, à la fois à un mode d'action spécifique à l'amiodarone et différent de l'effet β-bloquant et aussi à l'obtention d'un effet β-bloquant plus important avec des doses de β-bloqueur plus faibles que celles qui sont habituelles.

Ceci facilite la réduction des doses de β-bloqueur d'une part, et la réduction des doses d'amiodarone d'autre part. Ceci a également pour effet de limiter tout effet secondaire de l'association de l'amiodarone et du β-bloqueur. Ainsi, l'association amiodarone/β-bloqueur améliore la qualité de vie du malade qui présente une insuffisance coronaire en comparaison avec l'emploi d'un β-bloqueur seulement.

Deux facteurs importants ont une influence sur l'effet global de la qualité de vie des malades angineux : performances physiques lors d'exercice et dysfonctionnement sexuel. Ces deux paramètres sont affectés d'une manière beaucoup moins sévère statistiquement par cette association de médicaments aux doses utilisées qu'avec un β-bloqueur seul aux doses standards (P< O,001) dans une étude de qualité de vie effectuée simultanément avec l'étude clinique décrite ci-dessus.

On a étudié les effets de l'association amiodarone/β-bloqueur par administration IV. Ces études ont été programmées pour effectuer la en raison sur des groupes de 6 malades, les effets de doses progressivement croissantes d'amiodarone et de propanolol, un β-bloqueur ayant un effet clinique immédiat. Ces études ont été comparées aux effets d'un mélange propanolol/amiodarone contenant des doses progressivement croissantes de propanolol. On a trouvé :

1°- que l'administration de petites doses de propanolol entre 1 et 5 mg augmentait d'une manière très importante les effets cliniques de petites doses d'amiodarone.

2°- que les effets immédiats d'une injection rapide de l'association propanolol 1 mg et amiodarone 25 mg dépassaient les effets immédiats constatés avec 50 mg d'amiodarone et dépassaient les effets retardés de 4 mg de propanolol.

Sur la base de ces expériences, on a effectué le développement d'une composition contenant 1 mg de propanolol et 25 mg d'amiodarone qui a été trouvé manifestant une efficacité au moins aussi puissante que celle de 50 mg d'amiodarone sur certains paramètres et aussi puissante que 3 mg de propanolol sur les mêmes paramètres et correlé à une survenue moindre d'effets secondaires que celle des deux médicaments utilisés séparement aux doses efficaces cliniquement.

Les doses mentionnées ci-dessus de β-bloqueur qui potentialisent l'effet de l'amiodarone par administration IV, ont été trouvées au cours d'essais sur 2 groupes de 8 malades ayant un angor instable et ont montré une synergie avec l'amiodarone. En ce qui concerne les effets anti-ischémiques, on observe un effet beaucoup plus précoce sur certains paramètres de l'ischémie, consommation de l'oxygène par le myocarde et modification du segment ST que lorsque l'amiodarone est utilisée isolément. La dose d'amiodarone requise pour produire en 2 heures une diminution de 20 % dans l'élévation du segment ST est sensiblement réduite lorsque 1 mg de propanolol et 2 mg d'aténolol sont administrés d'une manière concomittante.

Une solution IV contenant 50 mg d'amiodarone, 1 mg de propanolol et 2 mg de dinitrate d'isosorbide a, en outre, été utilisée avec succès chez 20 malades présentant une insuffisance coronaire aiguë sévère sous forme de dose unique répétée chaque heure pendant 12 heures.

Une solution semblable contenant 5 mg d'acebutolol à la place du propanolol a été également utilisée chez 12 malades. Lorsque l'on compare ces résultats avec ceux de malades témoins comparables, les résultats obtenus avec cette nouvelle composition apparait extrêmement satisfaisante, en particulier avec une incidence moindre d'infarction du myocarde, d'arythmie sévère et de morts subites.

c) l'association amiodarone/β-bloqueur et un nitrate par administration iv a alors été étudiée.

les essais ont été effectués dans des cas d'insuffisance coronaire aiguë chez 5 malades présentant un infarctus du myocarde aigü en utilisant des doses groupées d'une composition contenant 1 mg de propanolol et 25 mg d'amiodarone en comparaison avec les effets d'une composition renfermant 2 mg de dinitrate d'isosorbide utilisée séparement. on a constaté l'existence d'une synergie entre le dinitrate d'isosorbide et la combinaison β-bloqueur/amiodarone. chez tous les sujets étudiés, l'administration de dinitrate d'isosorbide par voie iv (2 à 3 mg par heure) après administration préalable de propanolol et d'amiodarone a amené une réaction beaucoup plus grande de l'ischémie et, dans 2 cas, une disparition complète de l'ischémie à partir du moment où les 3 médicaments sont utilisés simultanément.

Dans une quatrième série d'essais, on a effectué des investigations sur les effets de l'administration orale des compositions pharmaceutiques mentionnées ci-dessus dans l'infarctus et dans le syndrôme infarctoide.

Une comparaison a été effectuée chez 8 malades atteints d'infarctus du myocarde et examinés dans les 30 à 60 minutes après le début de la douleur en étudiant les effets de l'administration soit :

. d'une association A : 20 mg de dinitrate d'isosorbide sous forme de comprimés associée à une prise sublinguale de 5 mg de dinitrate d'isosorbide,

. soit d'une association B : formée des préparations suivantes administrées par voie orale :
   a) amiodarone        400 mg
   b) aténolol        50 mg
   c) dinitrate d'isosorbide        20 mg
   d) dinitrate d'isosorbide        5 mg (par voie sublinguale)

On a constaté des effets bénéfiques nettement supérieurs mesurés par les indices électriques de l'ischémie et les indices habituels de la consommation d'oxygène avec l'association "B" qui, en outre, n'a entraîné aucun effet indésirable important.

Les essais qui précèdent immédiatement, ont été répétés en remplaçant les 50 mg d'aténolol par 100 mg d'acebutolol ou par 20 mg de propanolol. A nouveau, des effets bénéfiques semblables ont été observés avec cette association "B" qu'avec les effets de l'association "A" comme définie ci-dessus et évaluée selon les mêmes critères.

Il apparait donc que les effets bénéfiques observés dans les deux syndrômes cliniques (angor chronique, syndrôme infarctoide aigü) par l'administration buccale de l'association d'amiodarone/aténolol ou d'amiodarone/acebutolol ou d'amiodarone/propanolol démontrent la synergie des effets de l'amiodarone d'une part et les effets du β-bloqueur d'autre part.

Les effets bénéfiques de l'association d'amiodarone/acebutolol d'une part, de l'amiodarone/atenolol ou l'amiodarone/propanolol d'autre part peuvent être étendus à la classe générale des β-bloqueurs.

d) dans une cinquième série d'essais, les effets de la thérapeutique conservatrice de l'angine de poitrine chronique par une association simultanée d'amiodarone, d'un β-bloqueur et d'un dérivé nitré sous la forme d'une dose unique journalière ont été observés.

Des β-bloqueurs (aténolol, acébutolol, métoprolol, sotalol et tertatolol) ont été administrés chacun à 6 malades. Chez les mêmes sujets, aux mêmes périodes, les effets suivants ont été observés :
   1°- dans l'angor induit par l'exercice et les modifications de l'E.C.G induites par l'exercice
   2°- survenues journalières des symptômes angineux
   3°- effets secondaires
   4°- critères de qualité de vie à des intervalles de 3 semaines

On a administré une association β-bloqueur à faible dose plus de l'amiodarone plus un nitrate par voie orale plus un nitrate par voie sublinguale. L'effet obtenu a été comparé à celui de chaque médicament utilisé séparement et à celui d'une composition progressive partant d'un médicament et allant à 3 médicaments. Le dérivé nitré étant essayé sous ses deux formes -orale et sublinguale-, séparement et en mélange.

Ces essais ont démontré une nette supériorité de l'association des trois médicaments par rapport à la combinaison des deux composés et à un composé unique séparé. Cette supériorité a été déterminée à la fois sur les tests d'exercice et sur la survenue des symptômes. L'association des trois composés a entraîné une amélioration importante de la qualité de vie. Ces effets sont généralement obtenus avec tous les β-bloqueurs utilisés et avec les 3 types de nitrate utilisés, dinitrate d'isosorbide, nitro glycérine et mononitrate d'isosorbide.

## MODES D'ADMINISTRATION ET EMPLOIS THERAPEUTIQUES

**a)** Administration chronique par voie orale

Le traitement en principe utilisant les trois substances actives dans les nouvelles compositions pharmaceutiques sous une forme destinée à l'administration orale dans un rapport pondéral (poids approximatif) d'amiodarone/dinitrate d'isosorbide/aténolol de 15 : 2 : 5 et plus particulièrement d'une manière préférée dans le rapport 150 mg : 20 mg : 50 mg.

En présence d'une sensibilité aiguë aux effets des β-bloqueurs et principalement chez les sujets où le rythme cardiaque initial est long, ont peut envisager des doses de β-bloqueur plus faibles comme par exemple : 25 mg d'aténolol au lieu de 50 mg et à ce moment là, le rapport pondéral d'amiodarone/dinitrate d'isosorbide/aténolol devient 15 : 2: 2,5.

Dans les cas où il existe une hyper sensibilité aux β-bloqueurs et/ou une contre-indication aux β-bloqueurs, la composition utilisée fait partie d'une unité posologique de préférence 1 comprimé dans lesquels les principes actifs sont présents dans un rapport pondéral approximatif d'amiodarone par rapport au dinitrate d'isosorbide variant d'un rapport de 10 pour 1 à 5 pour 1 et de préférence de 7,5 pour 1 et plus particulièrement dans le rapport 150 mg/20 mg. On peut substituer au dinitrate d'isosorbide le trinitrate de glycéryle (nitro glycérine), le mononitrate d'isosorbide, le tétranitrate de pentaerythrytyle ou le tétranitrate d'érythrytyle.

**b)** Voie orale - Traitement d'urgence

Dans le traitement des urgences comme celui de l'insuffisance coronaire sévère, la composition préférée contient les principes actifs décrits ci-dessus dans une seule unité associés simultanément à l'administration d'un dérivé nitré à libération rapide ou résorption rapide notamment par voie sublinguale et de préférence sous la forme de nitro glycérine et de dinitrate d'isosorbide. Selon l'invention, cette composition fait partie d'une dose unitaire mais pour des raisons d'ordre pratique et de coût de développement, il est possible également de réaliser deux préparations unitaires que l'on utilisera, l'une étant destinée à l'administration par voie orale et l'autre par voie sublinguale, respiratoire ou transdermique. Lorsque l'on a constaté une hyper sensibilité aux β-bloqueurs, la forme préférée comprendra dans une unité d'administration l'amiodarone et le dérivé nitré, de préférence de l'amiodarone et du dinitrate d'isosorbide dans un rapport pondéral approximatif ou exact variant de 20 pour 1 à 5 pour 1 et d'une manière tout à fait préférée, dans le rapport de 400 à 20 mg. Cette dernière forme est réservée pour des épisodes d'insuffisance coronaire aiguë sévère et/ou d'arythmie sévère.

Les comprimés selon l'invention peuvent contenir par exemple de l'amiodarone, de l'acébutolol et du dinitrate d'isosorbide ou bien de l'amiodarone, de l'aténolol et du dinitrate d'isosorbide en incluant comme excipient du lactose, de l'amidon de maïs et de la polyvinyl povidone, du stéarate de magnésium, de mannitol, la carboxyméthyl cellulose sodique, du carboxyméthyl amidon sodique et de la silice colloïdale.

**c)** Administration par voie I.V

L'administration par la voie I.V apparait être la forme préférée d'administration des nouvelles compositions dans les cas d'insuffisance coronaire sévère, en particulier en cas d'infarctus constitué ou en phase de constitution, car c'est la forme la mieux adaptée au traitement du sujet. Cette forme d'administration peut être modulée en rapport avec l'évolution clinique et la susceptibilité individuelle. Elle comprend l'administration de 2 ou 3 composés et la composition idéale est celle qui permet l'administration des 3 composés.

Lorsque deux principes actifs sont administrés, par exemple l'amiodarone et le composé nitré en solution, un rapport variant de 100 : 1 à 5 : 1 et de préférence dans un rapport de 25 : 2 pour les doses unitaires et de 7 : 1 pour les doses d'entretien, est utilisé lorsqu'un β-bloqueur est employé. En outre le rapport amiodarone/β-bloqueur varie de 25 : 1 à 25 : 4 pour le propanolol, de 50 : 1 à 50 : 10 pour l'aténolol, de 25 : 2 à 25 : 10 pour l'acébutolol et de 25 : 2 à 25 : 15 pour le métoprolol.

Pendant les premières heures d'insuffisance coronaire aiguë, l'administration par voie parentérale de 2 à 6 mg/kg d'amiodarone et une dose de 2,5 à 7,5 mg par heure de dinitrate d'isosorbide apparait désirable. Une période de traitement de 12 heures est appropriée. La quantité d'amiodarone et de dinitrate d'isosorbide rendue nécessaire pour la limitation optimale et la prévention effective d'un infarctus est en moyenne de 400 à 50 mg respectivement administrés en 12 heures pour un malade de 70 kg. Les doses doivent être adaptées à l'évolution clinique en particulier en ce qui concerne les rapports liés aux quantités approximatives ou exactes d'amiodarone et de dinitrate d'isosorbide qui varient de 20 : 1 à 5 : 1 et de préférence 7 : 1 et d'une manière tout à fait préférée qui contiennent 700 mg d'amiodarone et 100 mg de dinitrate d'isosorbide administrés en 24 heures.

Les doses unitaires les mieux adaptées pour le traitement d'urgence d'une coronarite aiguë consistent, à la lumière des expérimentations effectuées, en ce que les deux substances se présentent sous le rapport optimum de 25 : à 25 : 2 et d'une manière tout à fait préférée dans un rapport de 25 : 2. La dose nécessaire est plus faible chez des sujets présentant une pression artérielle normale, une résistance artérielle systémique normale et chez ceux présentant une pression pulmonaire normale que chez ceux ayant une pression capillaire pulmonaire élevée, une pression artérielle élevée ou une résistance artériolaire ischémique élevée. De même, les doses efficaces et nécessaires sont moins importantes dans les cas d'infarctus inférieurs par rapport à ceux d'infarctus antérieurs dans les maladies cardiaques ischémiques de longue date et chez les sujets âgés par rapport aux maladies coronaires d'apparition récente ou chez les malades plus jeunes.

En outre, en présence d'insuffisance coronaire aiguë et de manière à prévenir le retour d'une insuffisance coronaire et d'obtenir un limitation optimale de la dimension de l'infarctus et la prévention efficace de l'infarction, on a constaté qu'une administration plus prolongée est désirable en fonction de l'intensité du syndrôme infarctoïde ou de l'angor instable. On a constaté que l'administration par voie IV peut être prolongée d'une manière optimale jusqu'à 12 heures en employant des doses de 400 mg d'amiodarone et 50 mg de dinitrate d'isosorbide ou prolongée jusqu'à 24 heures en employant une dose totale de 700 mg et 100 mg respectivement.

Lorsqu'une administration nécessitait en outre un β-bloqueur, le rapport préféré de l'amiodarone au β-bloqueur dans les formulations pour la voie IV est de 25 : 1 dans le cas du propanolol ou de 25 : 2 dans le cas de l'aténolol.

Les formes idéales de l'appareillage nécessaire pour l'administration des nouvelles compositions dans le traitement des urgences aiguës consistent en conséquence en :

1°- soit une seringue comportant 2 ou 3 compartiments qui contiennent les 2 ou 3 principes actifs, prête à l'emploi

2°- un kit contenant 2 ou 3 seringues prêtes à être fixé sur un dispositif de régulation comportant 3 ou 4 pistes pour lesquelles : l'une est fixée à une aiguille prête à l'emploi et les 2 ou 3 autres sont fixées à des seringues qui contiennent les principes actifs déjà dosés conformément au rapport amiodarone/dinitrate d'isosorbide variant de 25 : 1 à 100 : 1 pour une injection initiale immédiate pendant les 5 premières minutes de l'intervention médicale et dans un rapport d'environ 100 mg : 4 mg pour des injections rapides pendant les premières heures des syndrômes d'infarction. La composition peut comprendre un β-bloqueur par exemple l'aténolol en quantité déjà déterminée de 2 à 10 mg dans un compartiment.

Les compositions peuvent contenir un β-bloqueur autre que l'aténolol, l'acébutolol, le satolol et le métoprolol comme par exemple le tertratolol et le labetolol.

Les doses approximatives préférées du β-bloqueur administrées par voie IV destinées à remplacer 2 mg d'aténolol est dans le cas du propanolol d'1 mg, dans le cas du métoprolol 8 mg (injections lentes) et dans le cas de l'acébutolol 5 mg.

Les doses préférées de β-bloqueur qui peuvent remplacer 50 mg d'aténolol dans la composition sont comme suit :

- acébutolol ... environ 100 mg de produit sous forme à libération immédiate et 250 mg sous forme d'une préparation à libération prolongée
- métoprolol ... environ 100 mg
- tertatolol .. environ 2,2 mg.

Les compositions peuvent contenir un dérivé nitré autre que le dinitrate d'isosorbide ou la nitro glycérine qui ont été utilisés par l'inventeur dans cette composition.

Les compositions qui contiennent de la trinitrine et qui ont fait l'objet d'investigations sont semblables à celles décrites ci-dessus en remplaçant 2,5 à 7,5 mg par heure de dinitrate d'isosorbide par 15 à 300 mg par minute de nitro glycérine administrés par IV. Pour l'administration par voie orale, la composition est semblable à celle qui utilise le dinitrate d'isosorbide tout en remplaçant 20 mg de dinitrate d'isosorbide par 5 à 7,5 mg de nitro glycérine par voie orale. On peut également utiliser le mononitrate d'isosorbide, le tétranitrate d'érythrityle ou le tétranitrate de pentaerythrityle à des doses approximatives ou exactes de 10 à 20 mg, 2 à 10 mg et 40 à 100 mg respectivement à la place de 20 mg de dinitrate d'isosorbide.

Dans le traitement des insuffisances coronaires extrêmement sévères où la vie humaine est en cause, les formes finies préférées de l'invention sont des préparations liquides, adaptées pour l'administration par voie parentérale.

## EXEMPLE I

**COMPOSITION PHARMACEUTIQUE POUR L'ADMINISTRATION PAR VOIE IV**

Présentation : solution injectable dans des boîtes renfermant des flacons de 3 ml chacun
Hôpital avec unité de soins coronaires, mobile :
Présentation : boîte de 20 flacons

**COMPOSITION DE LA SOLUTION :**

dinitrate d'isosorbide          2 mg
amiodarone                      25 mg
propanolol                      1 mg
mannitol                        3 mg
glycine                         17,5 mg
chlorure de sodium              30 mg
acide acétique                  0,12 mg
eau q.s pour 3 ml
hydroxyde de sodium q.s pour pH7

EXEMPLE II

AUTRES PREPARATION POUR LA VOIE IV

La présentation est effectuée sous forme de flacons comme à l'exemple I.

COMPOSITION DE LA SOLUTION :

| | |
|---|---|
| dinitrate d'isosorbide | 2 mg |
| amiodarone | 25 mg |
| aténolol | 2 mg |
| mannitol | 3 mg |
| glycine | 17,5 mg |
| chlorure de sodium | 30 mg |
| acide acétique | 0,12 mg |
| eau q.s pour 3 ml | |
| hydroxyde de sodium q.s pH7 | |

EXEMPLE III

COMPOSITION PHARMACEUTIQUE SOUS FORME DE COMPRIMES

On a réalisé un comprimé renfermant les 3 principes actifs suivants dans les proportions ci-après :

| | |
|---|---|
| amiodarone ............................. | 150 mg |
| aténolol .............................. | 50 mg |
| dinitrate d'isosorbide ................. | 275 mg |

Autres constituants : Excipients

| | |
|---|---|
| lactose ..... | 275 mg |
| amidon de Maïs | 80 mg |
| povidone .... | 6 mg |
| stéarate de Mg | 11 mg |
| silice colloïdale | 2,5 mg |

EXEMPLE IV

CAPSULES A DEUX OU TROIS COMPARTIMENTS

1°- on a réalisé une capsule à 3 compartiments de façon à inclure les principes actifs suivants et les excipients inertes :

- un compartiment inclut le mononitrate d'isosorbide et est associé aux excipients inertes de manière à réaliser une libération prolongée.

| Substances actives (composition approximative) | Excipients inertes (composition approximative en mg) |
|---|---|
| . Chlorhydrate d'amiodarone 0,075 g | Lactose ............. 0,025<br>Amidon de Maïs ......<br>Povidone excipient... 0,002<br>Silice colloïdale ... 0,0009<br>Stéarate de magnésium 0,0017<br>Eau purifiée ........ 0,022 |
| . Chlorhydrate d'acébutolol 0,200 g | lactose ............. 0,15<br>Amidon de Maïs ...... 0,45<br>Povidone ............ 0,13<br>Stéarate de magnésium 0,004<br>Talc ................ 0,001<br>Aerosil 200 ........ 0,001 |
| . 5-mononitrate d'isosorbide 0,030 g | Lactose ............. 0,090<br>Micro granules de :<br>Saccharose ......... 0,033<br>Amidon de Maïs ...... 0,011<br>Polymère d'acide métha-crylique et d'esters d'acide méthacrylique<br>(Eudragit L) ....... 0,006<br>Gomme .............. 0,090 |

2°- une capsule à 2 compartiments :
- 1er compartiment : amiodarone 100 mg plus excipients inertes comme mentionnés ci-dessus et, soit 5 mg de nitro glycérine, soit 10 mg de dinitrate d'isosorbide

Excipients inertes : Talc .................. 0,009

Lactose .............. 275 mg
Amidon de Maïs ........ 80 mg
Povidone excipient .... 6 mg
Stéarate de magnésium . 11 mg
Silice colloïdale ..... 2,5 mg

- 2ème compartiment : acébutolol sous une forme à libération prolongée.

## EXEMPLE V

**COMPRIMES A LA FOIS POUR ADMINISTRATION SUBLINGUALE ET GASTRO-INTESTINALE**

dinitrate d'isosorbide :

```
– pour administration par voie sublinguale........... 2,5 mg
– pour l'opération gastro-intestinale .............. 25 mg


amiodarone ........................ 150 mg
métoprolol ........................  50 mg
excipients : lactose ............. 300 mg
              amidon de maïs ....... 100 mg
              povidone excipient ...   6 mg
              stéarate de magnésium   15 mg
              silice colloïdale ....  2,5 mg
```

## EXEMPLE VI

**COMPOSITION POUR ADMINISTRATION PAR VOIE ORALE POUR LE TRAITEMENT D'URGENCE**

```
dinitrate d'isosorbide ........      5 mg
(à libération sublinguale)


dinitrate d'isosorbide ........     15 mg
(à libération gastro-intestinale après absorption orale)


amiodarone .................... 400 mg
aténolol ......................  50 mg
excipients : lactose .......... 300 mg
              amidon de maïs .... 100 mg
              povidone excipient    6 mg
              stéarate de magnésium  15 mg
              sil ice colloïdale   2,5 mg
```

Dans les exemples 3 à 6 à la place d'aténolol, la composition peut être formée avec un autre β-bloqueur en particulier de l'acébutolol, du métoprolol, du sotalol, du tertatolol ou du propanolol. Une dose de 100 mg d'acébutolol sous la forme d'un modèle à libération immédiate ou 250 mg d'un modèle à libération lente peut remplacer 50 mg d'aténolol dans la composition et une dose de 1OO mg de métoprolol ou de 40 mg de sotalol ou de 2,5 mg de tertatolol ou de 20 mg de propanolol peuvent également remplacer 50 mg d'aténolol dans les compositions pour la voie orale.

## EXEMPLE VII

**SERINGUE PRETE A L'EMPLOI ET KIT AINSI QUE LES METHODES DE TRAITEMENT POUR LES CAS D'URGENCE**

En raison de la nécessité d'adapter les doses aux cas individuels et de prévenir les morts subites, un dispositif est utilisé qui comprend dans une seule unité :

1°- une seringue déjà prête contenant dans un compartiment une dose de 25 mg d'amiodarone pour injection unique et dans un autre compartiment, une dose de 100 mg d'amiodarone pour l'administration sur une durée variant de 2 à 5 minutes après la première injection, ainsi qu'un troisième compartiment contenant 200 mg d'amiodarone destinés à être administré après un délai de 15 à 60 minutes après la seconde injection.

2°- un compartiment de seringue contenant 2 mg de dinitrate d'isosorbide ainsi qu'un autre compartiment contenant 4 mg de dinitrate d'isosorbide

## EXEMPLE VIII

**SERINGUES PRE-REMPLIES CONTENANT LES COMPOSITIONS CI-APRES :**

1ère réalisation : une seringue pré-remplie à plusieurs réservoirs contenant dans des compartiments séparés pour une administration simultanée

a)
| dinitrate d'isosorbide | 2 mg |
| eau | 2 ml |
| acide acétique | 0,12 mg |
| glycine | 17,5 mg |
| mannitol | 3 mg |

b)
| amiodarone | 25 mg |
| alcool benzylique | 15 mg |
| polysorbate 80 | 50 mg |
| eau | 0,05 ml |

c)
| propanolol | 1 mg |
| acide citrique | 20 mg |
| eau | 1 ml |

2ème réalisation : une seringue pré-remplie contenant dans des départements séparés :

a)
| dinitrate d'isosorbide | 8 mg |
| acide acétique | 0,48 mg |
| glycine | 70 mg |
| mannitol | 12 mg |

b)
| amiodarone | 100 mg |
| alcool benzylique | 60 mg |
| polysorbate 80 | 200 mg |
| eau | 2 ml |

c)
| aténolol | 8 mg |
| acide citrique | 20 mg |
| eau | 1 ml |

La seringue peut être mise en mouvement par une micro pompe programmable de manière à éjecter les doses ajustées de la composition.

## EXEMPLE IX

**KIT POUR LE TRAITEMENT DE L'INSUFFISANCE CORONAIRE AIGUË**

Un dispositif ou un kit comprend une seringue décrite à l'Exemple I -1ère réalisation- et renferme à la place du dinitrate d'isosorbide pour la voie IV comme l'exemple 8 -2ème réalisation- ci-dessus, un paquet de nitro glycérine et/ou de nitrate d'isosorbide à 5 mg pour l'administration par voie sublingale et/ou un inhalateur d'un exemple de dérivé nitré comme le dinitrate d'isosorbide (dose de 1,5 mg) ou de nitro glycérine ou de mononitrate d'isosorbide.

La disponibilité de ce kit permet au médecin de démarrer immédiatement un traitement approprié pour prévenir la mort subite et pour limiter la taille de l'infarctus.

## EXEMPLE X

## EXEMPLE DE DOSAGES EQUIVALENTS ALTERNATIFS

Il est entendu, pour l'homme de l'art, que les différents dérivés nitrés et les β-bloqueurs utilisés dans la présente invention ont des activités différentes par unité de base. C'est pourquoi, pour une gamme donnée de dose d'amiodarone, les équivalents des autres principes actifs qui peuvent être utilisés dans les compositions et procédés selon la présente invention sont fournis ci-après :

| Nitrate | Dose par voie orale | Dose pharmaceutique | Entretien |
|---|---|---|---|
| dinitrate d'isosorbide | 10-20 mg | 2-10 mg | 2-50 mg |
| nitro glycérine | 2,5-10 mg | 0,1-2 mg | 15-150 g/mn = 0.9-9 mg |
| tétranitrate de pentaerythrityle | 10-100 mg | | |
| mononitrate d'isosorbide | 5-40 mg | | |
| tétranitrate d'érythrityle | 5-10 mg | | |

| β-bloqueurs | | | |
|---|---|---|---|
| propanolol | 20-40 mg | 1-5 mg | |
| sotalol | 40-80 mg | 2-40 mg | |
| acébutolol | 100-500 mg | 5-25 mg | |
| aténolol | 25-100 mg | 1-5 mg | |
| métoprolol | 25-100 mg | 1-5 mg | |
| pindolol | 5-10 mg | | |
| tertalol | 2,5-5 mg | | |
| Amiodarone | 40-400 mg | 25-100 mg | 0,5-20 mg/kg/j |

## EXEMPLE XI

## PROCEDE DE TRAITEMENT DE L'INSUFFISANCE CORONAIRE CHRONIQUE EN VUE D'AMELIORER LA QUALITE DE VIE ET D'EVITER LE RETOUR DE MORTS SUBITES

La sensibilité à un β-bloqueur doit être déterminé en premier lieu par une dose d'essai du β-bloqueur, par exemple 50 mg d'aténolol administrés par voie orale le 1er jour et d'aténolol à 100 mg administrés le second jour . L'absence de toute bradycardie d'une intensité inhabituelle, d'hypotension ou de blocage atrio-ventriculaire après administration du β-bloqueur autorise l'utilisation des compositions selon l'invention contenant les trois principes actifs.

Une des compositions selon la présente invention comme par exemple, l'exemple III est adminitrée deux fois par jour pendant 6 à 10 jours jusqu'à ce qu'on ait réalisé la diminution optimale du rythme cardiaque, c'est-à-dire de façon à réaliser un rythme cardiaque permanent entre 60 et 70.

Une dose est ainsi adaptée aux résultats obtenus et variera d'une manière habituelle entre 1/2 et 1 comprimé 1/2 par jour.

## EXEMPLE XII

## EXEMPLE D'UN PROCEDE DE TRAITEMENT DE L'INSUFFISANCE CORONAIRE AIGUE PRESENT DANS LE TRAITEMENT MYOCARDIAL ET DANS L'ANGOR INSTABLE, EN VUE DE PREVENIR LA SURVENUE D'UN INFARCTUS DU MYOCARDE, DE LIMITER SA TAILLE ET DE PREVENIR LES MORTS SUBITES

L'administration par voie orale de la composition de l'ensemble 6 peut être effectuée par un malade préalablement à l'arrivée de l'équipe médicale et aussi précocement que possible après l'apparition d'une douleur pré-cordiale.

L'administration est également effectuée par le médecin ou par l'équipe de réanimation de la composition de l'exemple I par voie IV. Des injections de doses répétées sont à administrer par l'équipe médicale en fonction des résultats obtenus sur la pression sanguine, le rythme cardiaque et d'autres symptômes ou signes cliniques. Ce traitement est continué par perfusion continue d'une composition qui contient 2 ou 3 principes actifs.

## Revendications

1. Utilisation d'une association d'Amiodarone et d'un vasodilatateur coronaire portant au moins un groupement nitré, pour la préparation d'un médicament destiné au traitement de l'insuffisance coronarienne et de ses complications cliniques, association qui présente un effet synergique vis-à-vis de ces troubles.

2. Utilisation selon la revendication 1° dans laquelle l'association comporte un agent beta-bloqueur.

3. Utilisation selon la revendication 1° ou la revendication 2° dans laquelle les principes actifs sont conditionnés séparement mais destinés à une administration conjointe, soit l'un après l'autre, soit deux d'abord, le dernier ensuite.

4. Utilisation selon l'une des revendications 1 à 3 dans lesquelles les principes actifs sont sous une forme adaptée à l'administration par voie orale, sublinguale, intra-veineuse, sous-cutanée ou respiratoire.

5. Utilisation selon l'une des revendications 1 à 4 caractérisée en ce que l'un des principes actifs est sous une forme administrable par voie intra-veineuse et l'une et/ou l'autre principe actif est sous une forme administrable par voie sublinguale, orale ou respiratoire.

6. Utilisation selon l'une des revendications 1 à 4 caractérisée en ce que les principes actifs sont présentés dans des compartiments multiples.

7. Utilisation selon l'une des revendications précédentes caractérisée en ce que l'amiodarone est présentée dans un compartiment, associée à au moins un autre principe actif et en ce que l'agent β-bloqueur est disposé dans un autre compartiment, présenté sous une forme à libération programmée.

8. Utilisation selon l'une des revendications précédentes dans laquelle l'amiodarone et au moins un des autres principes actifs sont dissouts dans un véhicule aqueux destiné à l'administration par voie parentérale.

**9.** Utilisation selon l'une des revendications précédentes dans laquelle l'amiodarone est présente sous forme de base, libre ou salifiée par un acide minéral ou organique.

**10.** Utilisation selon l'une des revendications précédentes dans laquelle le composé organique vasodilatateur coronaire est choisi dans le groupe constitué par le dinitrate d'isosorbide, un mononitrate d'isosorbide, le tétranitrate de pentaerythrityle et la trinitroglycérine.

**11.** Utilisation selon l'une des revendications 2 à 10° dans laquelle l'agent β-bloqueur est choisi dans le groupe constitué par l'acébutolol, le propanolol, le métoprolol, le tertatolol, le sotalol, l'aténolol et le pindolol.

**12.** Utilisation selon l'une des revendications précédentes dans laquelle le rapport pondéral entre l'amiodarone etle coronaro dilatateur nitré varie de 40 pour 4 à 40 pour 1,5.

**13.** Utilisation selon l'une des revendications 2 à 12° dans laquelle le rapport pondéral entre l'amiodarone et l'agent β-bloqueur varie de 100 à 10 à 100 à 300.

**14.** Utilisation selon l'une des revendications 2 à 13° dans laquelle l'agent β-bloqueur est présent à la fois sous une forme à libération immédiate et sous une forme à libération programmée.

**15.** Utilisation selon l'une des revendications 2 à 14° dans laquelle l'amiodarone et l'agent β-bloqueur se trouvent présents dans une forme pharmaceutique adaptée à l'administration par voie orale et le coronaro-dilatateur nitré sous une forme adaptée à l'administration sublinguale.

**16.** Utilisation selon l'une des revendications précédentes dans laquelle les principes actifs sont répartis dans une seringue à plusieurs compartiments sous forme de solutions prêtes à l'emploi.

**17.** Utilisation selon l'une des revendications 2 à 16° destinée à l'administration chronique par voie orale qui contient 150 mg d'amiodarone, 20 mg d'agent coronaro-dilatateur nitré et une quantité d'agent β-bloqueur autre que l'aténolol variant de 25 à 50 mg par prise unitaire.

**18.** Utilisation selon l'une des revendications 1 à 16° d'un médicament destiné au traitement des urgences cardiaques par voie orale qui consiste en ce que l'on emploie un médicament renfermant 400 mg d'amiodarone et 20 mg d'un agent coronaro-dilatateur nitré, par prise unitaire.

## Claims

**1.** Use of a combination of Amiodarone and a coronary vasodilator having at least one nitro group, for the production of a medicine intended for the treatment of coronary insufficiency and its clinical complications, combination which displays a synergistic effect against those disturbances.

**2.** Use according to claim 1 wherein the combination includes a β-blocking agent.

**3.** Use according to claim 1 or claim 2 wherein the active ingredients are separately packed but intended for a joint administration either one after the other, or two at first then the last one.

**4.** Use according to anyone of claims 1 to 3 wherein the active ingredients are in a form suitable for administration by oral, sublingual, intra-venous, subcutaneous or respiratory ways.

**5.** Use according to anyone of claims 1 to 4 wherein one of the active ingredients is in a form suitable for the administration per intra-venous way and the one and/or the other active ingredient is in a form suitable for administration per sublingual, oral or respiratory way.

**6.** Use according to anyone of claims 1 to 4 wherein the active ingredients are offered in multiple compartments.

**7.** Use according to any of the preceding claims wherein Amiodarone is offered in a compartment in conjunction with at least one other active ingredient and wherein the β-blocking agent is located in another compartment, offered in a sustained release form.

8. Use according to any of the preceding claims wherein Amiodarone and at least one of the other active ingredients are dissolved in an aqueous vehicle intended for administration per parenteral way.

9. Use according to any of the preceding claims wherein Amiodarone is present in the form of the base, free or salified with a mineral or organic acid.

10. Use according to any of the preceding claims wherein the organic coronary vaso-dilator is selected from the group consisting of Isosorbide dinitrate, Isosorbide mononitrate, pentaerythrityl tetranitrate and trinitroglycerol.

11. Use according to any of claims 2 to 10 wherein the β-blocking agent is selected from the group consisting of Acebutolol, Propanolol, Metoprolol, Tertatolol, Sotalol, Atenolol and Pindolol.

12. Use according to any of the preceding claims wherein the weight ratio between Amiodarone and the nitro coronaro-dilator ranges from 40 for 4 to 40 for 1,5.

13. Use according to any of the claims 2 to 12 wherein the weight ratio between Amiodarone and the β-blocking agent ranges from 100 for 10 to 100 for 300.

14. Use according to any of the claims 2 to 13 wherein the β-blocking agent is present both in an immediate release form and in a sustained release form.

15. Use according to any of the claims 2 to 14 wherein Amiodarone and the β-blocking agent are present in a pharmaceutical composition suitable for administration per oral way and the nitro coronaro-dilator in a form suitable for sublingual administration.

16. Use according to any of the preceding claims wherein the active ingredients are divided in a syringe with several compartments in the form of ready-to-use solution.

17. Use according to any of the claims 2 to 16 of a drug intended for chronic administration by oral way, which contains 150 mg Amiodarone, 20 mg of coronaro-dilator agent and an amount of a β-blocking agent other than Atenolol ranging from 25 to 50 mg per unit dosage.

18. Use according to any of claims 1 to 16 of a drug intended for the treatment of cardiac urgencies by oral way, which consists in that a medecine containing 400 mg Amiodarone and 20 mg of a nitro coronaro-dilator per unit dosage, is utilized.

**Patentansprüche**

1. Verwendung einer Kombination von Amiodaron und eines kranzgefässerweiternde Mittels, das mindenstens eine Nitrogruppe trägt, für die Bereitung eines fur die Behandlung des Kranzinsuffisenz und seiner klinischen Verwicklungen bestimmt, Arzneimittels, die eine synergischen Wirkung gegenüber diese Störungen aufweist.

2. Verwendung nach Anspruch 1 worin die Kombination eines Beta-blockierende Mittel einschliesst.

3. Verwendung nach Anspruch 1 oder Anspruche 2, worin die Wirkstoffe, allein aber für eine Mitwerwendung bestimmt sind, entweder nacheinander oder zwei zunächst dann der letzte, verpackt sind.

4. Verwendung nach eine der Ansprüche 1 bis 3, worin die Wirkstoffe in einer Form für per oralen Weg, sublingualen Weg, intravenösen, subkutanen oder respiratorischen Weg geeignet sind.

5. Verwendung nach einer der Ansprüche 1 bis 4, dadurch gekennzeichnet dass eine der Wirkstoffe unter einer Form der durch intravenöse Weg verabreichbar ist.

6. Verwendung nach einer der Ausprüche 1 bis 4, dadurch gezennzeichnet dass die Wirkstoffe in vielfältigen Abteilungen vorgelegt werden.

7. Verwendung nach einer der vorherigen Ansprüche, dadurch gekennzeichnet dass Amiodaron in eine Abteilung, mit mindenstens eine andere Wirkstoff vorgelegt wird und dass das Beta-blockierendes Mittel in eine andere Abteilung, und in einer Zubereitung mit protrahierte Freisetzung, angeordnet wird.

8. Verwendung nach einer der vorherigen Ansprüche worin Amiodarone und mindenstens eine der anderen Wirkstoffe in einem wässrigen für parenterale Verabreichung bestimmtes Medium, aufgelöst sind.

9. Verwendung nach einer der vorherigen Ansprüche worin Amiodaron in der Form von einer freie oder mit einer anorganischen oder organischen Säure versalzte Base, vorgelegt ist.

10. Verwendung nach einer der vorherigen Ansprüche worin die gefässerweiternde organische Verbindung aus der gruppe von Isosorbid dinitrate, Isosorbid mononitrate, Pentaerythrityl tetranitrate, un Trinitroglyzerin ausgewählt ist.

11. Verwendung nach einer der Ansprüche 2 bis 10 worin das Beta-blockierendes Mittel aus der gruppe von Acebutolol, Propanolol, Metoprolol, Tertatolol, Sotalol, Atenolol und Pindolol ausgewählt ist.

12. Verwendung nach einer der vorherigen Ansprüche worin das Gewichts verhältnis zwischen Amiodaron und der nitro kranzgefässerweiterndes Mittel von 40 für 4 bis 40 für 1,5 schwankt.

13. Verwendung nach einer der Ansprüche 2 bis 12 worin das Gewichtsverhältnis zwischen Amiodaron und das Beta-blockierendes Mittel von 100 zu 10 bis 100 zu 300 schwankt.

14. Verwendung nach einer der Ansprüche 2 bis 13 worin das Beta-blockierendes Mittel gleichzeitig in einer Form mit sofortigen Freisetzung und in einer Form mit protrahierte Freisetzung vorhanden ist.

15. Verwendung nach einer der Ansprüche 2 bis 14, worin Amiodarone und das Beta-blockierendes Mittel in einer pharmazeutische, für die orale Verabreichung, geeignete Zubereitung, anwesend sind und worin das nitro kranzgefässerweiterndes Mittel in einer Form die für die sublinguale Verabreichung geeignete ist, anwesend ist.

16. Verwendung nach der vorherigen Ansprüche, worin die Wirkstoffe in einer Spritze mit mehrfache Abteilungen, in der Form einer gebrauchsfertige Lösung verteilt sind.

17. Verwendung nach der Ansprüche 2 bis 16 eines Arzneimittels für chronische Abreichung durch oralen Weg, bestimmt, das 150 mg Amiodaron, 20 mg eines kranzgefässerweiterndes Mittel und eine Menge an ein Beta-blockierendes Mittel, das anderes als Atenolol ist, die sich zwischen 25 bis 50 mg per Einheitdosis erstreckt, enthält.

18. Verwendung nach der Ansprüche 1 bis 16 eines Arzneimittels das fur die Behandlung von herzlischen Dringlischkeiten, durch oralen Weg bestimmt ist die darin besteht dass das Arzneimittel das 400 mg Amiodaron und 20 mg eines nitro kranzgefässerweiterndes Mittel per Einheitdosis enthält, gebraucht ist.